# EUROPÄISCHE PATENTANMELDUNG

(11) **EP 1 477 142 A2**
(43) Veröffentlichungstag der Anmeldung: **17.11.2004**
(21) Anmeldenummer: 04011303.7
(22) Anmeldetag: 12.05.2004
(51) Int. Cl.: A61F 2/38

(54) **Kniegelenkendoprothese**

(30) Priorität: 31.12.2003 DE 10361770; 13.05.2003 DE 10321315
(71) Anmelder: Privelop AG, 53819 Neunkirchen-Seelscheid (DE)
(72) Erfinder: Schäfer, Björn, 53809 Ruppichteroth (DE); Kloss, Henning, 6373 Ennetbürgen (CH); Schütz, Peter, Dr., 68259 Mannheim (DE); Kloss, Hans, Peter, Dr., 64380 Rossdorf (DE)
(74) Vertreter: Arth, Hans-Lothar, Dr.

(57) **Zusammenfassung**

Die vorliegende Erfindung betrifft eine Kniegelenkendoprothese, welche aufgrund der Ausgestaltung der Radien der artikulierenden Oberflächen von Femurkomponente und Tibia-Onlay selbst bei einer seitlichen Beugebewegung der Femurkomponente um die anteriore-posteriore Achse die Kontaktfläche maximiert, wodurch eine optimale Lastverteilung erreicht und der Verschließ des Implantats minimiert wird. Ferner kann die Kniegelenkendoprothese eine derartige Lagerung des Tibia-Onlay auf der Tibiakomponente aufweisen, welche innerhalb bestimmter Grenzen eine Rotationsbewegung als auch eine Translationsbewegung des Tibia-Onlays auf der Tibiakomponente zulässt.

## Beschreibung

Die vorliegende Erfindung betrifft eine Kniegelenkendoprothese, welche aufgrund derselben Radien der artikulierenden Oberflächen von Femurkomponente und Tibia-Onlay selbst bei einer seitlichen Beugebewegung der Femurkomponente um die anteriore-posteriore Achse die Kontaktfläche maximiert, wodurch eine optimale Lastverteilung erreicht und der Verschleiß des Implantats minimiert wird.

Die deutsche Patentschrift DE 198 23 325 C1 offenbart ein Kniegelenkendoprothesensystem mit einem plattenförmigen Basisteil als Tibiakomponente. Auf diesem Basisteil lagert ein mit der Femurkomponente verbundenes Tibia-Onlay derart, dass eine Kippbewegung zwischen Tibia und Femur ermöglicht wird. DE 198 23 325 C1 verwendet herkömmliche Ausgestaltungen der Femurkomponente sowie daran angepasste gängige Ausgestaltungen der mit der Femurkomponente artikulierenden Oberflächen des Tibia-Onlays. Um die gewünschte Kippbewegung, d.h. eine seitliche Beugebewegung zu gewährleisten, wird die Femurkomponente kippbar auf dem Tibia-Onlay gelagert.

Aufgabe der vorliegenden Erfindung ist es, eine Kniegelenkendoprothese bereitzustellen, welche ein Maximum an anatomischer Kompatibilität erreicht und die Bewegungsfreiheitsgrade eines natürlichen Kniegelenks bestmöglichst imitiert und auch bei unter mechanischen Stressbedingungen auftretende punktuelle oder linienförmige Belastungsspitzen auf dem Tibia-Onlay gleichmäßig verteilt.

Diese Aufgabe wird durch die Bereitstellung einer Kniegelenkendoprothese gemäß Hauptanspruch gelöst. Weitere vorteilhafte Ausgestaltungen, Aspekte und Details der Erfindung ergeben sich aus den abhängigen Ansprüchen, der Beschreibung, den Beispielen und den Figuren.

Damit eine gelenkerhaltende Knieendoprothese ein Optimum an Wirksamkeit und Haltbarkeit erreichen kann, muss sie die anatomischen Bedingungen im Kniegelenk berücksichtigen und gleichzeitig die mechanische Funktion des Gelenkes sicherstellen, ohne dabei übermäßigem Verschleiß ausgesetzt zu sein.

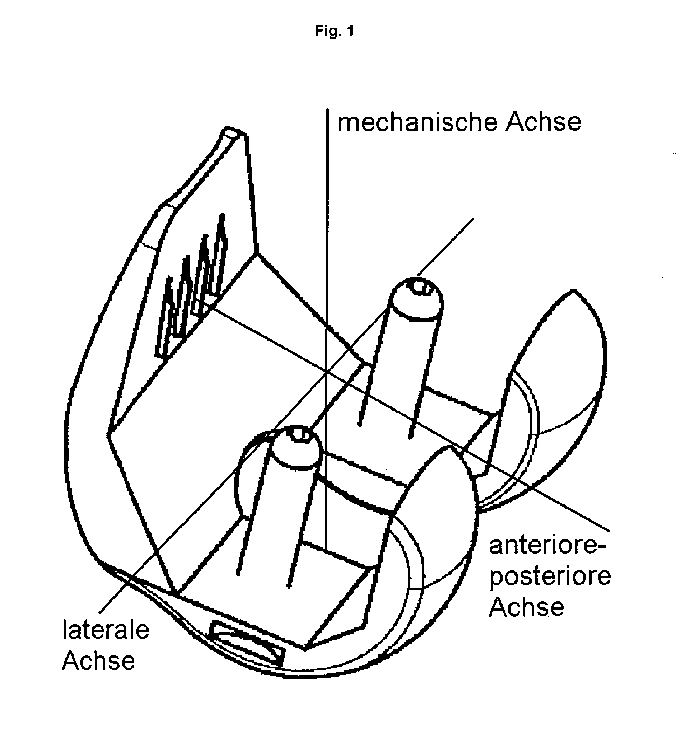

Ein natürliches Kniegelenk gewährt nicht nur eine Beugebewegung um die laterale Achse (Beugeachse), sondern auch eine geringe Beugebewegung senkrecht zur lateralen Achse um die anteriore-posteriore Achse sowie eine Rotationsbewegung um die mechanische (sagittale) Achse bzw. die anatomische Achse (s. Fig. 1).

Damit ein künstliches Kniegelenk diese komplexen Bewegungsfreiheitsgrade mit geringem Verschleiß erlauben kann, bedarf es einer besonderen, erfindungsgemäßen Ausgestaltung der artikulierenden Oberflächen der Kondylen der Femurkomponente als auch der mit der Femurkomponente artikulierenden Oberflächen des Tibia-Onlays.

Erfindungsgemäß wird diese Aufgabe durch die Bereitstellung einer Kniegelenkendoprothese gelöst, welche eine Femurkomponente mit zwei anterior (vorderseitig) durch ein Patellaschild starr miteinander verbundenen Kondylen und ein Tibia-Onlay umfasst, wobei die artikulierende Oberfläche einer jeden Kondyle der Femurkomponente sowie die mit der jeweiligen Kondyle artikulierende Oberfläche des Tibia-Onlays auf einem Kugelflächenausschnitt liegen und die beiden Kugelflächenausschnitte, auf denen die artikulierenden Oberflächen der beiden Kondylen liegen, denselben Radius aufweisen, wie die beiden Kugelflächenausschnitte, auf denen die mit der jeweiligen Kondyle artikulierenden Oberflächen des Tibia-Onlays liegen.

Unter "artikulierende Oberfläche" ist der Teil der Femuroberfläche sowie der Teil der Tibia-Onlay-Oberfläche zu verstehen, die miteinander in Berührung kommen können. Als Kontaktfläche soll die Fläche verstanden werden, wo bei einer bestimmten eingefrorenen Stellung der Kniegelenkendoprothese der Teil der artikulierenden Oberflächen der Kondylen der Femurkomponente mit dem Teil der artikulierenden Oberfläche des Tibia-Onlays in Berührung kommt.

Die artikulierende Oberfläche der Femurkomponente ist hingegen die gesamte Oberfläche der Femurkomponente, welche bei allen möglichen Stellungen der Kniegelenkendoprothese mit dem Tibia-Onlay in Berührung kommen kann.

Entsprechend ist die artikulierende Oberfläche des Tibia-Onlays die gesamte Oberfläche des Tibia-Onlays, welche bei sämtlichen möglichen Positionen der Femurkomponente mit der Femuroberfläche in Berührung kommen kann.

Es versteht sich, dass bei einer bestimmten Stellung der Kniegelenkendoprothese nicht die gesamte artikulierende Femuroberfläche mit der artikulierenden Oberfläche des Tibia-Onlays in Berührung kommen kann. Somit ist die Kontaktfläche immer nur eine Teilfläche der artikulierenden Femuroberfläche.

Bei einer Beugebewegung um die laterale Achse gleiten beide Kondylen der Femurkomponente gleichmäßig auf dem Tibia-Onlay. Um die Femurkomponente positionsstabil auf dem Tibia-Onlay zu lagern, weist das Tibia-Onlay zwei zur Aufnahme der Kondylen geeignete Senken, im folgenden Tibia-Senken genannt auf.

Diese beiden Tibia-Senken sind kugelförmig ausgestaltet, d.h. die artikulierende Oberfläche einer jeden Tibia-Senke liegt auf einem Ausschnitt einer Kugeloberfläche. Bevorzugt sind die Radien beider Tibia-Senken identisch, dies muss jedoch nicht immer zwingend der Fall sein. Zum Ausgleich von natürlichen Ungleichmäßigkeiten in der Knieregion können die beiden Tibia-Senken auch einen voneinander abweichenden Radius aufweisen.

Damit nun bei der besagten Beugebewegung bei jeder Stellung der Femurkomponente, d.h. bei jeder Stellung der Kondylen zum Tibia-Onlay dieselbe Lastverteilung auf dem Tibia-Onlay erreicht werden kann, sind die Kondylen derart ausgestaltet, dass sie mit ihrer Kontaktfläche exakt in die jeweilige Tibia-Senke passen.

Dies bedeutet, dass auch die artikulierende Oberfläche einer jeden Kondyle auf einem Ausschnitt einer Kugelfläche liegt, deren Radius mit dem entsprechenden Radius des Kugelflächenausschnitts identisch ist, auf dem die artikulierende Oberfläche einer jeden Tibia-Senke liegt.

In dem bevorzugten Fall, dass beide Tibia-Senken denselben Radius aufweisen, besitzen alle vier Kugelflächenausschnitte denselben Radius, nämlich der Kugelflächenausschnitt auf dem die artikulierende Oberfläche der einen Tibia-Senke liegt, der Kugelflächenausschnitt auf dem die artikulierende Oberfläche der anderen Tibia-Senke liegt, der Kugelflächenausschnitt auf dem die artikulierende Oberfläche der einen Kondyle liegt und der Kugelflächenausschnitt auf dem die artikulierende Oberfläche der anderen Kondyle liegt.

Sind die beiden Radien der Tibia-Senken unterschiedlich, so weist das gesamte System zwei Radien auf, einen für den Kugelflächenausschnitt der einen Tibia-Senke sowie der zugehörigen artikulierenden Kondyle und einen zweiten Radius für die andere Tibia-Senke und deren zugehörigen artikulierenden Kondyle. Bevorzugt weichen diese beiden Radien aber nicht mehr als 10%, insbesondere bevorzugt 5% voneinander ab.

Erfindungsgemäß ist die gesamte artikulierende Oberfläche beider Tibia-Senken bei einer Gleitbewegung beider Kondylen auf dem Tibia-Onlay mit ihrer gesamten Kontaktfläche identisch.

Bei dieser Gleitbewegung beider Kondylen auf dem Tibia-Onlay ist die artikulierende Oberfläche des Tibia-Onlays von der Implantatgröße abhängig. D.h., die Gesamtkontaktfläche beider Kondylen mit dem Tibia-Onlay liegt zwischen 900 und 1700 mm² je nach Ausführungsform des Implantats. Die kleineren Ausführungsformen weisen Kontaktflächen von 900 - 1100 mm² auf, die mittelgroßen Ausführungsformen von 1200 - 1400 mm² und die großen Ausführungsformen von 1600 - 1750 mm². Insbesondere liegen die Kontaktflächen bei steigender Größe der Ausführungsformen beginnend mit der kleinsten Variante zwischen 915 - 955 mm², bevorzugt 930 - 940 mm², insbesondere bevorzugt 936 mm², bzw. zwischen 1025 - 1065 mm², bevorzugt 1040 - 1050 mm², insbesondere bevorzugt 1046 mm², bzw. zwischen 1210 - 1250 mm², bevorzugt 1225 - 1235 mm², insbesondere bevorzugt 1230 mm², bzw. zwischen 1340 - 1380 mm², bevorzugt 1355 - 1365 mm², insbesondere bevorzugt 1362 mm² bzw. zwischen 1650 - 1690 mm², bevorzugt 1665 - 1675 mm², insbesondere bevorzugt 1672 mm².

Die Auflagefläche von Tibia-Onlay auf Tibiakomponente, d.h. die Kontaktfläche zwischen Tibia-Onlay und Tibiakomponente liegt zwischen 2000 mm² und 3900 mm². Bei den kleineren Ausführungsformen liegt diese Fläche im Bereich von 2100 - 2500 mm², bei den mittelgroßen Ausführungsformen im Bereich von 2750 - 3300 mm² und bei den großen Ausführungsformen im Bereich von 3700 - 3800 mm². Insbesondere liegen die Kontaktflächen bezogen auf die Auflagefläche von Tibia-Onlay auf Tibiakomponente bei steigender Größe der Ausführungsformen beginnend mit der kleinsten Variante zwischen 2110 - 2150 mm², bevorzugt 2125 - 2140 mm², insbesondere bevorzugt 2133 mm², bzw. zwischen 2440 - 2480 mm², bevorzugt 2455 - 2470 mm², insbesondere bevorzugt 2462 mm², bzw. zwischen 2820 - 2860 mm², bevorzugt 2835 - 2845 mm², insbesondere bevorzugt 2839 mm² bzw. zwischen 3220 - 3260 mm², bevorzugt 3235 - 3245 mm², insbesondere bevorzugt 3239 mm², bzw. zwischen 3725 - 3765 mm², bevorzugt 3740 - 3750 mm², insbesondere bevorzugt 3744 mm².

Diese Kontaktflächen beziehen sich bevorzugt auf Radien, welche größer als 20 mm sind und bevorzugt in dem Bereich von 20 - 40 mm liegen. Insbesondere bevorzugt sind Radien im Bereich von 22 bis 25 mm. Unterschreiten die Radien eine Länge von 20 mm, so können sich auch die oben beriebenen Kontaktflächen um den Bruchteil der Längenverkürzung verkleinern.

Die oben genannten absoluten Kontaktflächen von Tibia-Onlay mit beiden Kondylen der Femurkomponente und die Kontaktfläche von Tibia-Onlay und Tibiakomponente lassen sich in Relation zueinander setzen. Diese Relation, d.h. das Verhältnis von Kontaktfläche beider Kondylen mit Tibia-Onlay zu Kontaktfläche von Tibia-Onlay mit Tibiakomponente beträgt 0,4100 bis 0,4560, bevorzugt 0,4150 - 0,4510, weiter bevorzugt 0,4190 - 0,4470 und insbesondere bevorzugt 0,4200 - 0,4340.

Neben der gängigen Beugebewegung des Knies um die laterale Achse kann auch eine seitliche Beugebewegung um die anteriore-posteriore Achse um wenige Grad, in der Regel bis zu 8 Grad stattfinden.

Bei dieser seitlichen Beugebewegung hebt sich eine Kondyle aus der Tibia-Senke, wodurch die gesamte Kontaktfläche halbiert wird. Nun entspricht die gesamte Kontaktfläche nur noch der artikulierenden Oberfläche einer Tibia-Senke.

Um bei dieser seitlichen Beugebewegung weiterhin eine optimale Lastverteilung zu erreichen, sind die Kondylen derart ausgestaltet, dass die artikulierende Oberfläche der mit dem Tibia-Onlay in Kontakt stehenden Kondyle weiterhin auf dem besagten Ausschnitt der Kugelfläche liegt, die denselben Radius aufweist, wie der Ausschnitt der Kugeloberfläche, auf der die artikulierende Oberfläche der mit der einen Kondyle in Kontakt stehenden Tibia-Senke liegt.

Durch diese erfindungsgemäße Ausgestaltung der Kondylen und der Tibia-Senken halbiert sich bei einer seitlichen Beugebewegung, bei der sich eine Kondyle aus einer Tibia-Senke heraushebt, nur die gesamte Kontaktfläche. Bei den Ausführungsformen des Standes der Technik werden bei derartigen komplexen Bewegungen punktförmige bzw. linienförmige Belastungsspitzen von bis zu 500 kg erreicht, welche zu einem starken Verschleiß des Implantats, insbesondere des Tibia-Onlays führen. Die erfindungsgemäße Ausgestaltung vermeidet derartige Belastungsspitzen insbesondere bei komplexen Bewegungen, was die Langzeitstabilität des Implantats und damit die Lebensdauer des Implantats deutlich verlängert. Dies ist sehr wichtig, da der Ersatz eines defekten bzw. funktionsunfähigen Knieimplantats problematisch ist.

Somit wird selbst bei komplexen lateralen-anterioren oder lateralen-posterioren Beugebewegungen eine Kontaktfläche von mindestens 275 mm², bevorzugt mindestens 290 mm², weiter bevorzugt mindestens 305 mm², noch weiter bevorzugt mindestens 315 mm² und insbesondere bevorzugt mindestens 325 mm² aufrechterhalten.

Anders ausgedrückt besteht die Erfindung darin, dass die Radien der artikulierenden Femuroberflächen und die Radien der artikulierenden Oberflächen des Tibia-Onlays sowohl in sagittaler als auch in frontaler Ebene durchgängig ausgestaltet sind.

Damit nun die natürlichen Bewegungsfreiheitsgrade bestmöglichst imitiert werden können, umfasst die erfindungsgemäße Kniegelenkendoprothese des weiteren eine Tibiakomponente, wobei Tibiakomponente und Tibia-Onlay derart ausgestaltet sind, dass das Tibia-Onlay auf der Tibiakomponente Rotationsbewegungen ausführen kann.

Diese Rotationsbewegungen des Tibia-Onlays auf der Tibiakomponente finden um die mechanische Achse bzw. die anatomische Achse und in der durch die laterale Achse und die anteriore-posteriore Achse aufgespannten Ebene statt.

Eine weitere Anpassung der Kniegelenkendoprothese an die anatomischen Verhältnisse bei den komplexen Bewegungsabläufen wird erfindungsgemäß dadurch erreicht, dass das Tibia-Onlay derart auf der Tibiakomponente gelagert ist, dass es entlang der lateralen Achse und/oder der anterioren-posterioren Achse Translationsbewegungen ausführen kann.

Eine derartige Lagerung des Tibia-Onlays auf der Tibiakomponente wird beispielsweise durch ein Befestigungsmittel erreicht. Als Befestigungsmittel kommen Zapfen, Auswölbungen, Halterungen, Wulst, erhöhte Ränder, Stifte und dergleichen sowie andere denkbare Mittel zur Beschränkung der Translationsbewegung von Tibia-Onlay auf der Tibiakomponente in Frage, welche bevorzugt auf der Tibiakomponente angebracht sind.

Die Tibiakomponente kann einen bevorzugt zentriert angebrachten Stift aufweisen, der sich in Richtung der mechanischen und/oder anatomischen Achse erstreckt. Bevorzugt weist dieser Stift einen Durchmesser von 3 mm bis 25 mm, weiter bevorzugt von 7 - 20 mm, noch weiter bevorzugt von 9 - 15 mm und insbesondere bevorzugt von 11 - 12 mm und eine Höhe von 3 mm bis 8 mm, bevorzugt von 4 - 7 mm, weiter bevorzugt von 5 - 6,5 mm und insbesondere bevorzugt von 5,9 - 6,1 mm auf. Ferner besitzt ein solcher Stift bevorzugt eine Zylinderform oder Kegelform, wobei jedoch generell ellipsoide Formen Verwendung finden können. Die Zylinderform ist bevorzugt. Bei der Verwendung von unsymmetrischen Formen oder anderen Formen als der Zylinderform, z.B. Kegelform, Stiftform mit wechselnden Radien, Pyramidenform, Nagelform, Stecknadelform, Halbkugelform und dergleichen sind die oben angegebenen Radien auf den Bereicht der halben Höhe des Befestigungsmittel zu beziehen. Dieser Stift bzw. dieses Befestigungsmittel sollte weitgehend zentriert auf der Tibiakomponente angebracht werden.

Das Tibia-Onlay weist erfindungsgemäß eine zur Aufnahme dieses Stiftes bzw. des Befestigungsmittels geeignete Aussparung auf, wobei diese Aussparung bevorzugt einen größeren Durchmesser als die des Stiftes aufweist. Eine derartige Aussparung ist bevorzugt O-förmig bis ellipsoid, kann aber auch kreisförmig ausgestaltet sein.
Bei der O-förmigen oder ellipsoiden Ausgestaltung ist der Radius in lateraler Richtung kleiner als der Radius in anteriorer-posteriorer Richtung.

Wählt man den Durchmesser dieser Aussparung nur geringfügig größer als den Durchmesser des Stiftes so wird die Translationsbewegung fast vollständig unterbunden und nur die Rotationsbewegung von Tibia-Onlay auf der Tibiakomponente ist möglich.

Bevorzugt hat der Radius der Aussparung in anteriorer-posteriorer Richtung die 1,0- bis 3,0-fache Länge des Radius des Stiftes. Der Radius der Aussparung in lateraler Richtung ist gleich groß oder bis zum 1,5-fachen größer, als der Radius des Stiftes.

Aufgrund der größeren Ausgestaltung der Aussparung im Tibia-Onlay im Vergleich zum Stift der Tibiakomponente, kann dieser Stift sich in den durch die Aussparung festgelegten Grenzen bzw. das Tibia-Onlay sich innerhalb dieser Grenzen translatorisch und natürlich auch rotatorisch auf der Tibiakomponente bewegen.

Durch die erfindungsgemäße Ausgestaltung dieses Befestigungsmittels und der Aussparung können Translationsbewegungen von bis zu 10 mm in anteriorer-posteriorer Richtung stattfinden. Dies bedeutet, daß das Tibia-Onlay auf der Tibiakomponente 10 mm von der einen Extremposition zur anderen Extremposition in anteriorer-posteriorer Richtung bewegt werden kann bzw. sich bewegen kann. Bevorzugt beträgt diese Strecke 7 mm, weiter bevorzugt maximal 6 mm und insbesondere bevorzugt maximal 5 mm.

Die Translationsbewegung in lateraler Richtung wird entweder gar nicht zugelassen oder nur in einem deutlich geringeren Maße, als die Bewegung in anteriorer-posteriorer Richtung. Die laterale Translationsbewegung ist bis maximal 5 mm möglich, bevorzugt bis zu maximal 4 mm, weiter bevorzugt bis zu maximal 3 mm und insbesondere bevorzugt bis zu maximal 2 mm zusätzlich zur Translationsbewegung in anteriorer-posteriorer Richtung möglich.

Ein Befestigungsmittel in Form eines weitgehend auf der Tibiakomponente zentriert angebrachten Stiftes schränkt die Rotationsbewegung des Tibia-Onlays auf der Tibiakomponente hingegen nicht ein. Die Rotationsbewegung um die mechanische oder anatomische Achse wird jedoch durch die natürlichen Bedingungen und/oder durch weitere Befestigungsmittel festgelegt. Derartige Befestigungsmittel sind bevorzugt auf der Tibiakomponente angebracht. Rotationsbewegungen von bis zu 10 Grand in beide Richtungen ausgehend von einer zentrierten Lage lässt die erfindungsgemäße Kniegelenkendoprothese zu. Es hat sich gezeigt, das es bevorzugt ist, die freie Rotation von Tibia-Onlay auf der Tibiakomponente nicht durch weitere Befestigungsmittels einzuschränken bzw. zu limitieren. Die anatomischen Gegebenheiten genügen vollends, eine Rotation in benötigen Masse zu erlauben bzw. in einem darüber hinaus gehenden Masse zu beschränken bzw. zu unterbinden.

Das Befestigungsmittel kann nicht nur aus einem Stift, Zapfen, Flansch oder ähnlichem bestehen, sondern auch zwei oder mehr dieser Befestigungsmittel umfassen.

Eine bevorzugte Ausführungsform der erfindungsgemäßen Kniegelenkendoprothese umfasst eine Tibiakomponente, welche auf der dem Tibia-Onlay zugewandten Oberfläche einen weitgehend zentral angebrachten Stift aufweist. Das zugehörige Tibia-Onlay weist eine auf der der Tibiakomponente zugewandten Seite angebrachte Aussparung zur Aufnahme dieses Stiftes auf. Ist diese Aussparung nicht kreisförmig sondern länglich, kreisbahnförmig wie eine Laufbahn im Stadion oder oval, so besitzt sie zwei Radien bzw. Durchmesser. Dieser Radius bzw. Durchmesser dieser Aussparung in lateraler und/oder anteriorer-posteriorer Richtung ist vorzugsweise größer als der Radius des Stiftes der Tibiakomponente. Bevorzugt ist der Radius bzw. Durchmesser dieser Aussparung in anteriorer-posteriorer Richtung größer als der Radius des Stiftes.

Ferner ist bevorzugt, wenn die Aussparung im Tibia-Onlay nicht durchgehend ist, d.h. das Tibia-Onlay nicht vollständig in Richtung der mechanischen bzw. anatomischen Achse durchbohrt wird. Die Aussparung auf der der Tibiakomponente zugewandten Seite des Tibia-Onlays ist von der der Femurkomponente zugewandten Seite des Tibia-Onlays nicht zu erkennen. Zudem ist die Aussparung im Tibia-Onlay nicht derart, dass sie von der Seite entlang der lateralen und/oder anterioren-posterioren Achse von aussen sichtbar wäre. Diese Aussparung durchbricht weder entlang der mechanischen bzw. anatomischen Achse, noch in Richtung der lateralen oder anterioren-posterioren Achse das Tibia-Onlay und führt nur zu einer Öffnung, welche aus Richtung der der Tibiakomponente zugewandten Seite zugänglich ist. Dadurch wird möglichst wenig Material vom Tibia-Onlay entfernt, wodurch die mechanische Belastbarkeit des Tibia-Onlays erhöht und seine Lebensdauer verlängert wird.

Eine weitere bevorzugte Ausführungsform umfasst zwei Stifte, welche auf der Tibiakomponente angebracht sind. Das Tibia-Onlay weist dementsprechend zwei Aussparungen auf, welche im Vergleich zum Durchmesser eines Stiftes einen größeren Durchmesser besitzen. Dadurch kann sich das Tibia-Onlay innerhalb der Aussparungen translatorisch frei um die Stifte bewegen, wobei die Translationsbewegung als auch die Rotationsbewegung um die mechanische oder anatomische Achse im Rahmen der Aussparungen möglich ist. Bei dieser Ausgestaltung ist eine theoretisch mögliche freie Rotation um 360 Grad nicht mehr ausführbar.

Anstelle von zwei Stiften können auch drei oder mehr verwendet werden, welche in der Regel mit gleichmäßigen Abständen auf der Tibiakomponente angebracht werden. Ferner können anstelle von Stiften auch seitliche Halterungen vorgesehen werden. Dabei wird die durch die seitlich an der Tibiakomponente befestigten Halterungen begrenzte Bodenfläche der Tibiakomponente größer ausgestaltet, als die aufliegende Bodenfläche des Tibia-Onlay, so dass das Tibia-Onlay Translations- und/oder Rotationsbewegungen auf der Tibiakomponente ausführen kann und zwar im Rahmen der seitlichen Halterungen.

Insbesondere bevorzugt bleibt die Ausführungsform, welche eine weitgehend auf der Tibiakomponente zentriert angebrachte Erhebung aufweist. Diese Erhebung ist vorzugsweise in Form eines Stiftes oder Zapfens oder auch in Form einer halbkugelförmigen, ovalen oder ellipsoiden Erhebung ausgebildet. Derartige Ausführungsformen weisen gegenüber den Varianten mit Mehrstift- oder Mehrpunktlagerung den Vorteil auf, dass das Tibia-Onlay eine entsprechende Aussparung zur Aufnahme des Stiftes, Zapfens oder der halbkugelförmigen, ovalen oder ellipsoiden Erhebung dort aufweist, wo eine geringe mechanische Belastung besteht. Das Tibia-Onlay ist im Vergleich zur Femurkomponente und zur Tibiakomponente der größten mechanischen Belastung und dem größten Verschleiß ausgesetzt. Aus diesem Grunde muss das Tibia-Onlay eine Mindestdicke aufweisen und sollte nach Möglichkeit vollständig massiv ausgebildet sein, d.h. keinerlei Aussparungen aufweisen. Da jedoch zur Ermöglichung der Translationsbewegungen von Tibia-Onlay auf der Tibiakomponente eine Aussparung zur Ausnahme des Befestigungsmittels erforderlich ist, sollte diese Aussparung an der Stelle der geringsten mechanischen Belastung vorgesehen werden. Die Stelle einer geringen Belastung befindet sich beim Tibia-Onlay weitgehend in zentrierter Position zwischen den beiden Tibia-Senken. Da Mehrstift- oder Mehrpunktlagerungen immer zumindest zwei Aussparungen im Tibia-Onlay verlangen, ist es bei diesen Ausführungsformen schwierig, Aussparungen in mechanisch nur gering belasteten Bereichen des Tibia-Onlays vorzusehen.

Durch die erfindungsgemäße Ausgestaltung der Kniegelenkendoprothese kann bei einer Beugebewegung des Kniegelenks neben der Gleitbewegung der Kondylen der Femurkomponente in den Tibia-Senken des Tibia-Onlays auch eine eingeschränkte Translations- und Rotationsbewegung des Tibia-Onlays auf der Tibiakomponente stattfinden. Diese komplexen Bewegungsmöglichkeiten imitieren die natürliche Bewegung bestmöglichst und sorgen bei jeder Stellung der Kniegelenkendoprothese für optimale Lastverteilung.

Ferner kann das Tibia-Onlay neben der erfindungsgemäßen Ausgestaltung der Tibai-Senken vorzugsweise zusätzlich einen antero-lateralen Flansch aufweisen.

Aufgrund der erfindungsgemäßen Ausgestaltung der artikulierenden Oberfläche der Femurkomponente sowie der artikulierenden Oberfläche des Tibia-Onlays erreicht man eine maximale Kongruenz von Femurkomponente zum korrespondierenden Tibia-Onlay. Daher ist die Flächenpressung auf dem Tibia-Onlay immer annähernd gleich und auf die größte mögliche Fläche verteilt.

Das führt dazu, dass selbst unter mechanischen Stressbedingungen punktuelle Belastungsspitzen auf dem Tibia-Onlay (meistens einem PE-Zwischenstück) vermieden werden, wodurch dessen Verschleiß besonders effektiv gemindert wird.

Ferner besteht das Tibia-Onlay vorzugsweise aus einem harten Kunststoff, bevorzugt Polyethylen und insbesondere Ultra High Molecular Weight Polyethylene (UHMWPE). Die Bezeichnung "ultrahochmolekulares Polyethylen" ist nicht eindeutig. Als HDPE (high density PE) gilt derzeit ein PE mit einer Molmasse von unter 200.000 g/mol. Nach DIN ISO 11542 ist PE mit einer Schmelze-Massefließrate von unter 0,1 g/10 min als UHMWPE definiert (wobei dies einer Molmasse von über 106 g/mol entspräche), nach der ASTM D 4020 liegt die Grenze bei 3,1*106 g/mol. Die angegebene, mittlere Molmasse heutigen UHMWPEs liegt, je nach Hersteller und verwendeter Messmethodik zwischen 3,5*106 und 107g/mol.

Bei einer weiteren bevorzugten Ausführungsform der Kniegelenkendoprothese besteht das Tibia-Onlay nicht aus PE sondern auch aus Titan, wobei das Titangrundmaterial wiederum bevorzugt mit einer keramischen Schicht und insbesondere bevorzugt mit Ti-Nb-N beschichtet ist. Bei dieser Ausführungsform ergibt sich eine sogenannte "hart-hart"-Paarung, d.h. sowohl für die Femurkomponente, insbesondere die Oberfläche der artikulierenden Kondylen als auch das Tibia-Onlay, insbesondere die artikulierende Oberfläche des Tibia-Onlays wurden extrem harte Materialien verwendet.

Das Tibia-Onlay ist auf der Tibiakomponente der Kniegelenkendoprothese frei gelagert, so dass sowohl eine rotatorische als auch eine translatorische Bewegung innerhalb des Gelenksystems ermöglicht wird, wie oben beschrieben. Dadurch werden der erfindungsgemäßen Kniegelenkendoprothese alle Bewegungsfreiheitsgrade des natürlichen Gelenks verliehen und sind in dem erfindungsgemäßen künstlichen Gelenksystem simultan möglich, ohne dass verschleißanfällige Scharniere eingebaut werden müssten. Durch diese Ausgestaltung wird insbesondere die Drehbewegung um die mechanische Achse sowie um die anatomische Achse entlang des Knochenverlaufs mit optimaler Lastverteilung ermöglicht.

Die Femurkomponente und/oder die Tibiakomponente der Kniegelenkendoprothese können zementiert oder zementfrei in den Knochen implantiert werden, wobei die zementfreie Implantation bevorzugt ist.

Der Körper der Femurkomponente als auch der Tibiakomponente kann aus den gängigen Materialien geformt sein. Er besteht bevorzugt aus einem Edelstahl, einer Legierung auf Kobalt-Basis, Titan oder einer Legierung auf Titan-Basis. Insbesondere bevorzugt ist die Verwendung von Titan, welches ferner bevorzugt zur Verhinderung des Metallatom- bzw. Metallionenaustritts mit einer Schutzschicht, beispielsweise einer keramischen Beschichtung überzogen ist.

Die Verwendung von Titan gegenüber dem Einsatz von bekannten Stahl- und Edelstahlmaterialien biete den Vorteil eines besseren Verwachsens des Implantats mit dem Knochen, da Stahl- und Edelstahlmaterialien aufgrund ihrer bio-physikalischen Eigenschaften nicht fest mit dem Knochen verwachsen und in den meisten Fällen einer Einzementierung des Implantats bedürfen. Zudem sind Patienten mit allergischen Reaktionen von der Verwendung von Implantaten aus Stahl- oder Edelstahlmaterialien ausgeschlossen.

Titan als Grundmaterial der erfindungsgemäßen Femur- und Tibiakomponente ist biologisch inert, verwächst daher fest im Knochen, kann zementfrei verankert werden und ist nicht allergen. Neben Titan können auch andere Metalle wie beispielsweise Stahl oder Titanlegierungen wie beispielsweise Ti-6Al-4V, Ti-Nb-Ta-Zr, Ti-Al6-Nb7 (nach ISO 5832-11) oder Ti-29Nb-13Ta-4.6Zr erfindungsgemäß Verwendung finden.

Die Femurkomponente und/oder die Tibiakomponente der Kniegelenkendoprothese sind vorzugsweise mit einer metallischen oder keramischen Beschichtung überzogen, die eine variable Anzahl der einzelnen Schichten oder eine unterschiedliche Schichtdicke aufweisen kann. Keramische Beschichtungen umfassen beispielsweise Nitride, Carbide und Phosphide von bevorzugt Halbmetallen und Metallen bzw. Metalllegierungen. Beispiele für keramische Beschichtungen sind Bornitride, Titan-Niob-Nitrid, Titan-Calcium-Phosphid (Ti-Ca-P), Cr-Al-N, Ti-Al-N, Cr-N, TiAIN-CrN, Ti-Al-C, Cr-C, TiAlC-CrC, Zr-Hf-N, Ti-Hf-C-N, Si-C-N-Ti, Si-C-N, DLC (diamond like carbon). Als Beschichtung wird ferner vorzugsweise eine keramische Schicht aus Titan-Niob-Nitrid (Ti-Nb-N) aufgebracht.

Insbesondere ist es vorteilhaft, wenn die artikulierende Oberfläche der Femurkomponente der Kniegelenkendoprothese mit Titan-Niob-Nitrid (Ti-Nb-N) beschichtet ist.

Diese keramische Beschichtung der insbesondere artikulierenden Implantatoberflächen hat eine Härte, die um ein Vielfaches höher ist, als dien der meisten herkömmlichen Knieprothesen.

Die Kniegelenkendoprothesen des Standes der Technik bieten nicht die Bewegungsfreiheitsgrade wie ein natürliches Kniegelenk, da in den Kopplungsmechanismen zur Verbindung der Implantatbausteine im Patienten eine Wiederherstellung der Gelenkfunktion nur begrenzt erreicht wird.

Ferner weisen die herkömmlichen Kniegelenkendoprothesen eine limitierte Oberflächenqualität der Implantate auf, was zu Abrieb am Polyethylenzwischenstück (Tibia-Zwischenstück) führt. Neben dem daraus resultierenden Verschleiß führen die Abriebpartikel zu Knochen auflösenden und Infekt induzierenden Reaktionen. Dieser Abrieb bzw. Verschleiß wird zudem durch suboptimale Oberflächengestaltung, d.h. ungleiche Lastverteilungsverhältnisse begünstigt, die innerhalb von wenigen Jahren zum Verschleiß des künstlichen Gelenkes führen können.

Ferner sind die erfindungsgemäßen Implantate derart ausgestaltet, dass sie als Implantat sowohl für ein rechtes als auch linkes Kniegelenk einsetzbar sind. Das bedeutet, dass Tibiakomponente, Tibia-Onlay und insbesondere die Femurkomponente sowohl für das linke als auch das rechte Knie einsetzbar sind.

Ermöglicht wird dies insbesondere durch die erfindungsgemäße Ausgestaltung des Patellagleitlagers der Femurkomponente. Die Konvexität der Patella entspricht in ihrem Radius der Konkavität zwischen den Kondylenerhebungen, so dass sie links wie rechts einsetzbar sind. Aufgrund der spiegelsymmetrischen Ausgestaltung von Femurkomponente, Tibia-Onlay und Tibiakomponente erübrigt sich das Problem, für rechte und linke Knieimplantate unterschiedliche Ausführungsformen, sogenannte anatomische Varianten bereitstellen zu müssen. Dies erleichtert die Anwendung und vermindert Lagerhaltungskosten.

### Figurenbeschreibung

- Figur 1: zeigt die Femurkomponente mit den drei Rotationsachsen, der mechanischen Achse, der lateralen Achse sowie der anteriorenposterioren Achse,

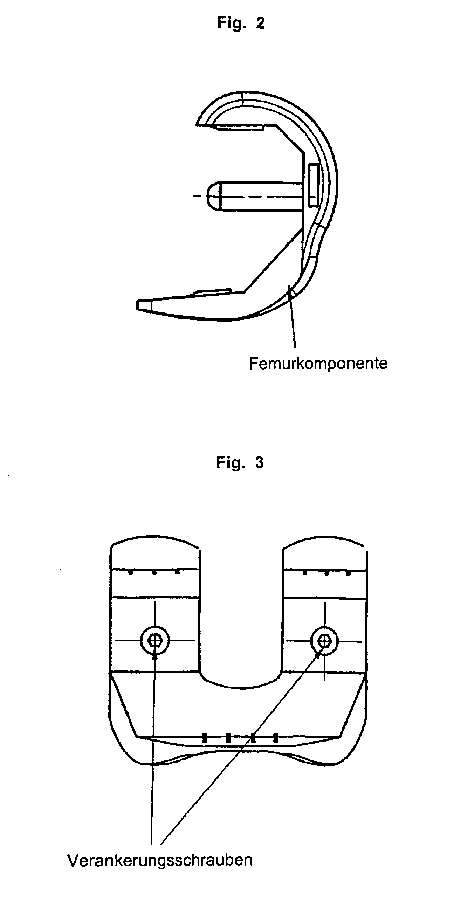

- Figur 2: zeigt eine Femurkomponente von medial bzw. lateral betrachtet (Seitenansicht),
- Figur 3: zeigt eine Femurkomponente von transversal betrachtet (Draufsicht),
- Figur 4: zeigt eine Femurkomponente von dorsal betrachtet (Rückansicht),
- Figur 5: zeigt eine Femurkomponente von medial bzw. lateral betrachtet (Seitenansicht),
- Figur 6: zeigt die Femurkomponente ohne die drei Rotationsachsen,
- Figur 7: zeigt eine Tibiakomponente aus frontaler Betrachtungsrichtung,
- Figur 8: zeigt eine Tibiakomponente in dimetrischer Darstellung,
- Figur 9: zeigt ein Tibia-Onlay in dimetrischer Darstellung,
- Figur 10: zeigt ein Tibia-Onlay betrachtet von der Bodenseite, die Aussparung im Tibia-Onlay ist gestrichelt gezeichnet und als durchgehende kreisförmige Linie ist das Befestigungsmittel der Tibiakomponente dargestellt. Es ist zu erkennen, dass das Befestigungsmittel der Tibiakomponente einen gewissen Bewegungsspielraum innerhalb der Aussparung im Tibia-Onlay hat.
- Figur 11: zeigt die Femurkomponente mit den artikulierenden Oberflächen der Kondylen. Die eingezeichneten Radien sollen verdeutlichen, dass die artikulierende Oberfläche einer jeden Kondyle auf einem Kugelflächenausschnitt liegt. Im gezeigten Fall sind die beiden Radien der Kugelflächenausschnitte, auf denen die artikulierenden Oberflächen einer jeden Kondyle liegen, identisch.
- Figur 12: zeigt das Tibia-Onlay mit den Tibia-Senken. Die gestrichelten Linien in den Tibia-Senken symbolisieren konzentrische Kreise, zu denen die Radien R derselben Länge führen, so dass verdeutlicht wird, dass die Tibia-Senken einen Teil einer Kugelfläche sind und sich dadurch ein Kugelflächenausschnitt als Kontaktfläche mit den Kondylen der Femurkomponente ergibt.

### Beispiele

Bevorzugte Ausführungsformen der erfindungsgemäßen Kniegelenkendoprothese werden nun anhand der Beispiele diskutiert, wobei zu berücksichtigen ist, dass die diskutierten Beispiele vorteilhafte Ausgestaltungen der Erfindung wiedergeben, jedoch den Schutzumfang nicht auf diese Ausführungsformen beschränkten.

### Beispiel 1

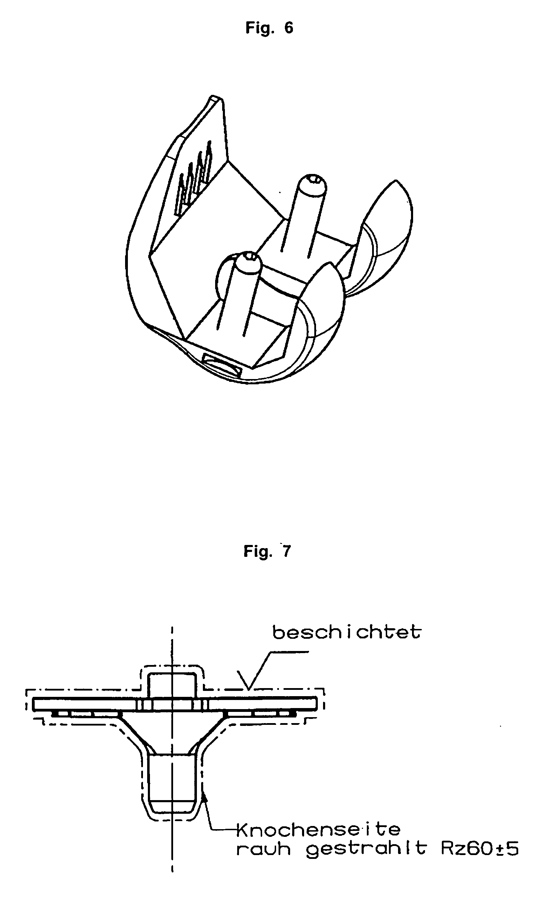

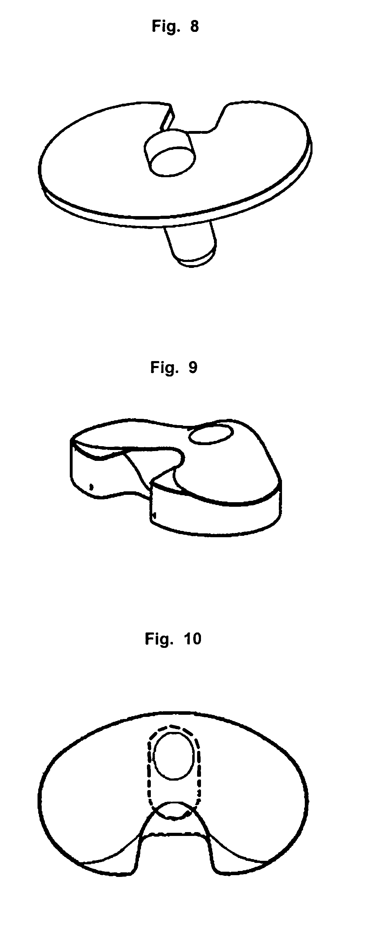

Eine Ausführungsform einer erfindungsgemäßen Kniegelenkendoprothese besteht aus einer Femurkomponente wie in Fig. 6 gezeigt, einer Tibiakomponente, wie in Fig. 7 dargestellt und einem Tibia-Onlay wie in Fig. 9 offenbart.

Die Kniegelenkendoprothese weist eine Größe auf, welche zur Implantation bei erwachsenen männlichen oder weiblichen Personen von 20 bis 80 Jahren und 1,7 - 2,2 m Größe geeignet ist. Kleinere Ausführungsformen der in Beispiel 1 beschriebenen Kniegelenkendoprothese sind durch einen Fachmann ohne Probleme herzustellen. Bei diesen kleineren Ausführungsformen können die Kontaktflächen insbesondere zwischen Kondylen und Tibia-Senken aber auch zwischen Tibia-Onlay und Tibiakomponente entsprechend der Größe der kleineren Ausführungsformen entsprechend kleiner ausfallen. Gleiches gilt für die oben angegebenen Werte für die Translationsbewegungen in lateraler als auch anteriorer-posteriorer Richtung.

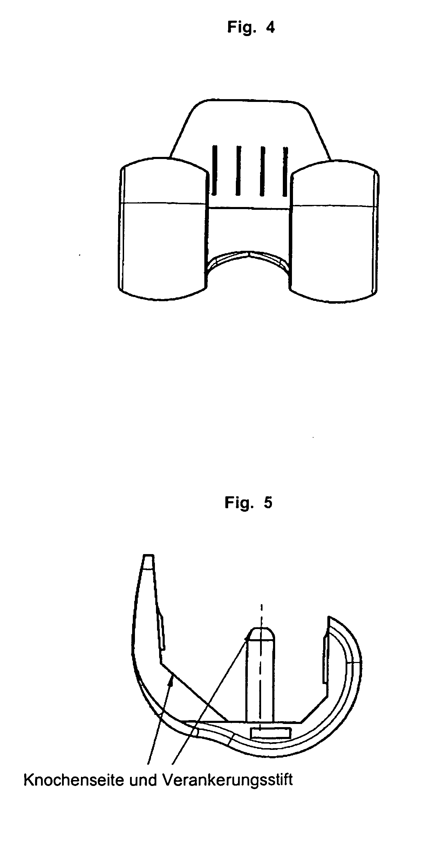

Die Femurkomponente besteht aus in der Medizintechnik verwendetem Titan. Patellaschild und Rotationsstift wie in Fig. 5 gezeigt sind herkömmlich ausgestaltet. Die dem Knochen zugewandte Oberfläche der Femurkomponente ist rauh, so dass ein Einwachsen bzw. Anwachsen von Knochenzellen ermöglicht wird. Die Rauhigkeit Rz beträgt ca. 60 ± 5 µm. Die artikulierenden Oberflächen der Kondylen sind mit einer keramischen Schicht aus Ti-Nb-N beschichtet. Die Schichtdicke beträgt mehrere µm.

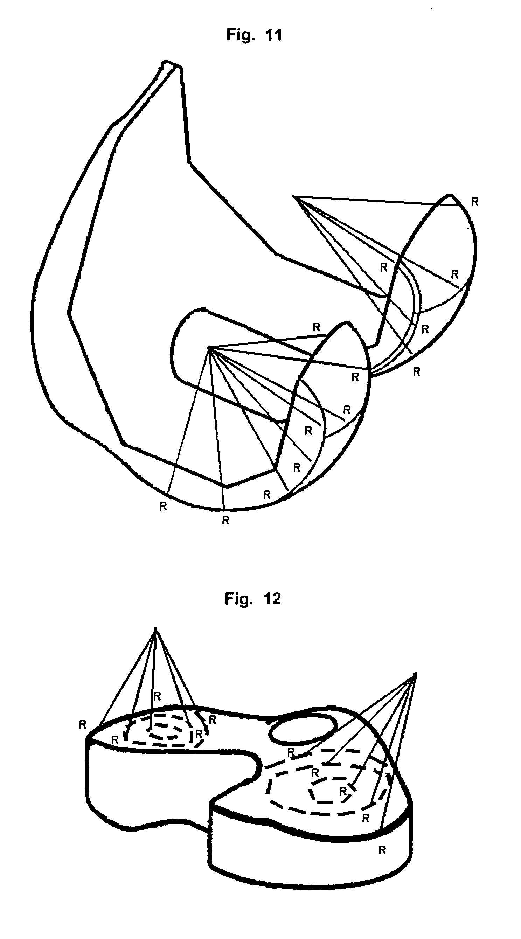

Die artikulierende Oberfläche einer jeden Kondyle liegt auf einem Kugelflächenausschnitt mit einem Radius von R = 25 mm wie in Fig. 11 verdeutlicht.

Die Tibiakomponente besteht ebenfalls aus Titan und weist eine Form wie in Fig. 8 gezeigt auf. Die dem Knochen zugewandte Oberfläche der Tibiakomponente ist rauh ausgestaltet mit einer Rauhigkeit Rz von ca. 60 ± 5 µm wie in Fig. 7 veranschaulicht. Die Auflagefläche für das Tibia-Onlay ist mit einer keramischen Beschichtung aus Ti-Nb-N beschichtet. Die Schichtdicke beträgt 5 - 8 µm.

Wie Fig. 8 verdeutlicht, ist die dem Tibia-Onlay zugewandte Oberfläche der Tibiakomponente planar, bis auf den mittig angeordneten Stift. Dieser Stift weist eine zylindrische Form mit einer Höhe von 6 mm und einem Durchmesser von 4,8 mm auf. Auch der Stift ist mit einer keramischen Beschichtung aus Ti-Nb-N versehen.

Das Tibia-Onlay weist eine Gestalt wie in Fig. 9 dargestellt auf. Das Tibia-Onlay besteht aus UHMWPE. Beide Tibia-Senken weisen dieselben Radien wie die artikulierenden Oberflächen der beiden Kondylen auf. Diese wird durch Fig. 12 verdeutlicht, welche die Tibia-Senken durch konzentrische gestrichelt gezeichnete Kreise darstellt, wobei der Radius zu einem jeden dieser konzentrischen Kreise gleich ist und mit R bezeichnet wird. R beträgt, wie bei dem Kugelflächenausschnitt, auf dem die artikulierende Oberfläche einer jeweiligen Kondyle liegt, 25 mm. Die Kondylen passen somit optimal in die Tibia-Senken und die artikulierende Oberfläche der Tibia-Senken entspricht der gesamten Kontaktfläche, welche ca. 650 mm² beträgt.

Ferner weist das Tibia-Onlay auf seiner der Tibiakomponente zugewandten Seite eine Aussparung auf, welche in Fig. 10 durch die gestrichelte Linie dargestellt wird. Diese Aussparung ist für die Aufnahme des Stiftes der Tibiakomponente vorgesehen. Dieser Stift wird in Figur 10 durch die durchgezogene Kreislinie symbolisiert.

Wie Fig. 10 zu entnehmen ist, ist die Aussparung in lateraler Richtung nur geringfügig größer als der Durchmesser des Stiftes. Durchmesser des Stiftes der Tibiakomponente beträgt 4,8 mm. Durchmesser der Aussparung im Tibia-Onlay beträgt 6,8 mm, so dass aus der zentrierten Position heraus, der Stift in lateraler Richtung jeweils 1,0 mm bewegt werden kann, bzw. das Tibia-Onlay auf der Tibiakomponente in lateraler Richtung jeweils 1,0 mm verschoben werden kann. Somit beträgt die Maximalbewegung in lateraler Richtung von einer Extremposition zur anderen 2,0 mm.

In anteriorer-posteriorer Richtung weist die Aussparung einen deutlich größeren Durchmesser als in lateraler Richtung auf. Der Durchmesser in anteriorer-posteriorer Richtung hat die 2,25-fache Länge wie der Durchmesser des Stiftes.

Der Durchmesser der Aussparung in anteriorer-posteriorer Richtung beträgt 10,8 mm. Somit ist die Aussparung im Tibia-Onlay 6 mm länger als der Durchmesser des Stiftes der Tibiakomponente.

Ausgehend von einer zentrierten Position kann sich das Tibia-Onlay auf der Tibiakomponente 3 mm in posteriorer Richtung als auch 3 mm in anteriorer Richtung translatorisch bewegen bzw. maximal 6 mm von einer Extremposition zur anderen Extremposition zurücklegen.

Insgesamt steht dem Tibia-Onlay auf der Tibiakomponente eine ovale Bewegungsfläche für Translationsbewegungen zur Verfügung, nämlich 2 mm Bewegungsfreiraum in lateraler Richtung und zusätzlich 6 mm Bewegungsfreiraum in anteriorer-posteriorer Richtung.

Eine Rotation von bis zu maximalen 20 Grad von einer Extremposition zur anderen bzw. von 10 Grad in beide Rotationsrichtungen ausgehend von einer zentrierten Position sind zusätzlich zu den Translationsbewegungen möglich.

Somit erlaubt die erfindungsgemäße Ausführungsform Bewegungsfreiheitsgrade wie bei einem natürlichen Kniegelenk, wobei auch bei komplexen Bewegungen durch die aufeinanderliegenden Kugelflächen von Kondylen und Tibia-Senken Belastungsspitzen auf dem Tibia-Onlay vermieden werden.

### Beispiel 2

Eine weitere Ausführungsform einer erfindungsgemäßen Kniegelenkendoprothese ist dreiteilig aus einer Femurkomponente, einer Tibiakomponente und einem Tibia-Onlay aufgebaut.

Die Kniegelenkendoprothese weist eine Größe auf, welche zur Implantation bei Personen von 1,5 - 1,9 m Größe geeignet ist. Kleinere als auch größere Ausführungsformen der in Beispiel 2 beschriebenen Kniegelenkendoprothese sind durch einen Fachmann ohne Probleme herzustellen. Bei den größenmäßig veränderten Ausführungsformen können Größe der Kontaktflächen sowie Ausmaß der Bewegungsmöglichkeiten entsprechend angepaßt sein.

Die Femurkomponente besteht aus in der Medizintechnik verwendetem Titan. Patellaschild und Rotationsstift wie in Fig. 5 gezeigt sind herkömmlich ausgestaltet. Die dem Knochen zugewandte Oberfläche der Femurkomponente ist rauh, so dass ein Einwachsen bzw. Anwachsen von Knochenzellen ermöglicht wird. Die Rauhigkeit Rz beträgt ca. 60 ± 5 µm. Die artikulierenden Oberflächen der Kondylen sind mit einer keramischen Schicht aus Ti-Nb-N beschichtet. Die Schichtdicke beträgt mehrere µm.

Die artikulierende Oberfläche einer jeden Kondyle liegt auf einem Kugelflächenausschnitt mit einem Radius von R = 22 mm wie in Fig. 11 verdeutlicht.

Die Tibiakomponente besteht ebenfalls aus Titan. Die dem Knochen zugewandte Oberfläche der Tibiakomponente ist rauh ausgestaltet mit einer Rauhigkeit Rz von ca. 60 ± 5 µm. Die Auflagefläche für das Tibia-Onlay ist mit einer keramischen Beschichtung aus Ti-Nb-N beschichtet. Die Schichtdicke beträgt ca. 5 µm.

Auf der dem Tibia-Onlay zugewandten Seite der Tibiakomponente sind zwei zylindrische Stifte seitlich mit gleichem Abstand zum Rand angeordnet. Der eine Stift befindet sich unter oder am Rande der einen Tibia-Senke während der andere Stift die äquivalente Position unter bzw. am Rande der anderen Tibia-Senke einnimmt. Beide Stifte weisen eine Höhe von 4 mm und einen Durchmesser von 4 mm auf. Beide Stifte bestehen aus Titan sind mit einer keramischen Beschichtung aus Ti-Nb-N versehen.

Das Tibia-Onlay weist von oben betrachtet eine Gestalt wie in Fig. 9 dargestellt auf. Das Tibia-Onlay besteht auch aus Titan und ist vollständig mit einer Schicht aus Ti-Nb-N überzogen. Beide Tibia-Senken weisen dieselben Radien wie die artikulierenden Oberflächen der beiden Kondylen auf (s. Fig. 12). Der Radius beträgt 22 mm. Aus fertigungstechnischen Gründen können die Radien um wenige zehntel Millimeter voneinander abweichen. Derartige Abweichungen der erfindungsgemäß als dieselben Radien bezeichnet. Die gesamte Kontaktfläche beträgt ca. 635 mm².

Ferner weist das Tibia-Onlay auf seiner der Tibiakomponente zugewandten Seite zwei Aussparungen zur Aufnahme der beiden auf der Tibiakomponente angebrachten Stiften auf. Beide Aussparungen sind kreisförmig und weisen einen Durchmesser von 6 mm auf, so dass ausgehend von einer zentrierten Position sich das Tibia-Onlay auf der Tibiakomponente in sämtliche Richtungen 1 mm bewegen kann bzw. 2 mm von einer Extremposition in eine andere zurücklegen kann.

Die beiden Stifte beschränken zudem die Rotationsbewegung auf ca. 6 - 7 Grad in beide Richtungen ausgehend von einer zentrierten Position bzw. auf eine absolute Rotation von 12 - 14 Grad.

### Beispiel 3

Die Femurkomponente ist wie in Beispiel 1 oder 2 beschrieben ausgestaltet.

Die Tibiakomponente besteht aus Titan mit einer Ti-Nb-N Beschichtung auf der artikulierenden Oberfläche. Die dem Tibia-Onlay zugewandte beschichtete Oberfläche der Tibiakomponente weist als Befestigungsmittel keine Stifte sondern einen Wulst am Rand, d.h. einen erhöhten Rand auf. Dieser Wulst kann durchgängig ausgestaltet sein oder unterbrochen.

Das Tibia-Onlay besteht aus Titan und ist auf seinen artikulierenden Oberflächen mit einer Beschichtung von Ti-Nb-N versehen. Die auf der Tibiakomponente aufliegende Grundfläche des Tibia-Onlays ist kleiner als die Fläche der Tibiakomponente, welche durch den erhöhten Rand begrenzt wird.

Somit hat das Tibia-Onlay einen gewissen Bewegungsspielraum innerhalb der durch den erhöhten Rand festgelegten Grenzen. In lateraler Richtung kann sich das Tibia-Onlay aus einer zentrierten Position heraus 1,5 mm in beide Richtungen, d.h. absolut 3,0 mm translatorisch bewegen.

In anteriorer-posteriorer Richtung kann sich das Tibia-Onlay 2,5 mm nach hinten bzw. nach vorne oder insgesamt eine Strecke von 5,0 mm von einer Extremposition zur anderen zurücklegen.

Der erhöhte Rand beschränkt die Rotationsbewegung des Tibia-Onlays auf der Tibiakomponente auf ca. 7 - 8 Grad.

## Patentansprüche

1. Kniegelenkendoprothese umfassend eine Femurkomponente mit zwei anterior durch ein Patellaschild starr miteinander verbundenen Kondylen und ein Tibia-Onlay, wobei die artikulierende Oberfläche einer jeden Kondyle der Femurkomponente sowie die mit der jeweiligen Kondyle artikulierende Oberfläche des Tibia-Onlays auf einem Kugelflächenausschnitt liegen und die beiden Kugelflächenausschnitte, auf denen die artikulierenden Oberflächen der beiden Kondylen liegen, denselben Radius aufweisen, wie die beiden Kugelflächenausschnitte, auf denen die mit der jeweiligen Kondyle artikulierenden Oberflächen des Tibia-Onlays liegen.

2. Kniegelenkendoprothese nach Anspruch 1 des weiteren umfassend eine Tibiakomponente, wobei das Tibia-Onlay derart auf der Tibiakomponente gelagert ist, dass es Rotationsbewegungen um die mechanische Achse ausführen kann.

3. Kniegelenkendoprothese nach Anspruch 1 oder 2 des weiteren umfassend eine Tibiakomponente, wobei das Tibia-Onlay derart auf der Tibiakomponente gelagert ist, dass es entlang der lateralen Achse und/oder der anterioren-posterioren Achse Translationsbewegungen ausführen kann.

4. Kniegelenkendoprothese nach einem der Ansprüche 2 oder 3, wobei die Tibiakomponente auf der dem Tibia-Onlay zugewandten Oberfläche einen weitgehend zentral angebrachten Stift und das Tibia-Onlay eine auf der der Tibiakomponente zugewandten Seite angebrachte Aussparung zur Aufnahme des Stiftes aufweist und der Radius dieser Aussparung in lateraler und/oder anteriorer-posteriorer Richtung größer als der Radius des Stiftes der Tibiakomponente ist.

5. Kniegelenkendoprothese nach einem der Ansprüche 1 - 4, wobei im Falle des Kontakts beider Kondylen mit dem Tibia-Onlay das Verhältnis von Kontaktfläche beider Kondylen mit Tibia-Onlay zu Kontaktfläche von Tibia-Onlay mit Tibiakomponente 0,4100 bis 0,4560 beträgt.

6. Kniegelenkendoprothese gemäß eines der Ansprüche 1 - 5, wobei die Femurkomponente und/oder die Tibiakomponente und/oder das Tibia-Onlay aus Titan und/oder einer Titanlegierung bestehen.

7. Kniegelenkendoprothese gemäß eines der Ansprüche 1 - 6, wobei die Femurkomponente und/oder die Tibiakomponente und/oder das Tibia-Onlay mit einer keramischen Beschichtung überzogen sind.

8. Kniegelenkendoprothese gemäß eines der Ansprüche 1 - 7, wobei die artikulierenden Oberflächen der Femurkomponente und die artikulierenden Oberflächen der Tibiakomponente und im Falle der Verwendung von Titan oder Titanlegierungen für das Tibia-Onlay auch die artikulierenden Oberflächen des Tibia-Onlays mit einer keramischen Beschichtung überzogen sind.

9. Kniegelenkendoprothese gemäß Anspruch 7 oder 8, wobei es sich bei der keramischen Beschichtung um eine Beschichtung aus Titan-Niob-Nitrid (Ti-Nb-N) handelt.

10. Kniegelenkendoprothese gemäß eines der Ansprüche 1 - 9, wobei die Femurkomponente und die Tibiakomponente zementfrei in den Knochen implantiert werden können.
